# FASCICULE DE BREVET EUROPEEN

(11) **EP 0 732 114 B1**
(45) Date de publication et mention de la délivrance du brevet: **23.08.2000**
(21) Numéro de dépôt: 96400490.7
(22) Date de dépôt: 08.03.1996
(51) Int. Cl.: A61M 5/162, A61J 1/05

(54) **Dispositif de perforation et ensemble de raccordement pour système d'alimentation par voie entérale et procédé de raccordement**
Perforationsvorrichtung und Anschluss für ein enterales Ernährungssystem und Verfahren für das Anschliessen
Perforation device and connecting assembly for an enteral alimentation system and connecting procedure

(30) Priorité: 13.03.1995 FR 9502881
(43) Date de publication de la demande: 18.09.1996
(73) Titulaire: SOCIETE DES PRODUITS NESTLE S.A., 1800 Vevey (CH)
(72) Inventeur: Bourguignon, Michel, 14740 Lasson (FR); Larrain, Ignaccio, 1028 Traverenges (CH)
(74) Mandataire: Casalonga, Axel

(56) Documents cités:
- EP-A- 0 476 386
- DE-A- 4 318 101

## Description

La présente invention concerne d'une manière générale les systèmes d'alimentation par voie entérale, et plus particulièrement, un ensemble de raccordement destiné à mettre en communication l'intérieur d'un conteneur scellé avec une tubulure d'alimentation pour l'alimentation par voie entérale d'un patient.

A la suite d'une opération, ou d'une manière plus générale lorsqu'une personne n'est plus à même de s'alimenter elle-même naturellement, il est souvent nécessaire d'apporter à cette personne les aliments nécessaires directement par voie entérale. Dans les systèmes d'alimentation par voie entérale, les aliments, sous forme d'un liquide nutritif, sont contenus dans un flacon scellé, généralement rigide, qui, au moment de l'utilisation, doit être raccordé et mis en communication avec une tubulure d'alimentation par voie entérale.

Un tel raccordement doit pouvoir s'effectuer dans des conditions d'asepsie évitant toute contamination du liquide nutritif contenu dans le flacon, et doit être fiable, c'est-à-dire qu'il ne doit pas présenter de fuite ni se reboucher en cours d'utilisation. En outre, ces systèmes d'alimentation par voie entérale sont généralement utilisés dans un environnement médical dans lequel on trouve d'autres systèmes de traitement du patient tels que, plus particulièrement, des systèmes d'injection par voie parentérale ou intraveineuse et il est important que lors de la mise en place du système d'alimentation par voie entérale, l'opérateur ne puisse pas, par inadvertance, raccorder le flacon contenant le liquide nutritif à un système d'injection par voie intraveineuse.

D'autre part, on a jusqu'à présent utilisé comme conteneur pour le liquide nutritif des flacons rigides, scellés au moyen d'une capsule que l'opérateur perce avant de raccorder le flacon à la tubulure d'alimentation. Un tel processus où le flacon est ouvert avant le raccordement entraîne un risque de contamination du liquide nutritif qu'il serait souhaitable d'éviter. Il serait également souhaitable de pouvoir utiliser à la place de flacons rigides des poches souples pour contenir le liquide nutritif analogues à celles utilisées pour le stockage des produits sanguins utilisés pour les transfusions.

Un dispositif selon le préambule de la revendication 1 est décrit dans le document EP-A-0 476 386.

La présente invention a donc pour objet un dispositif de perforation, en particulier pour un système d'alimentation par voie entérale remédiant aux inconvénients ci-dessus.

La présente invention a également pour objet un ensemble de raccordement pour un système d'alimentation par voie entérale remédiant aux inconvénients ci-dessus.

Plus particulièrement, la présente invention a pour objet un ensemble de raccordement qui permette d'utiliser comme conteneur soit un flacon rigide scellé soit une poche souple scellée.

En outre l'invention a pour objet un tel ensemble de raccordement qui assure que le conteneur contenant le liquide nutritif soit mis en communication avec la tubulure d'alimentation par voie entérale dans des conditions d'asepsie optimales.

L'invention a encore pour objet un tel ensemble de raccordement qui, lorsque l'on utilise comme conteneur une poche souple scellée évite tout risque de rebouchage du système d'alimentation par voie entérale par du matériau souple de la poche.

L'invention a aussi pour objet un ensemble de raccordement qui empêche toute erreur de la part de l'opérateur et en particulier qui empêche toute possibilité de raccordement à un système d'injection par voie intraveineuse.

Selon l'invention, on atteint les objectifs ci-dessus en réalisant un dispositif de perforation qui comporte:
- un élément de perforation formé d'une base tubulaire ayant un évidement ouvert à une première extrémité pour recevoir et maintenir une extrémité de la tubulure d'alimentation, une partie en forme d'aiguille faisant saillie à partir d'une seconde extrémité de ladite base et ayant un canal longitudinal central communiquant avec ledit évidement et débouchant à une extrémité libre de la partie en forme d'aiguille, ladite partie en forme d'aiguille ayant une section droite substantiellement circulaire et au moins une portion située à proximité de ladite base tubulaire dont la section droite comporte au moins une partie en retrait par rapport au cercle circonscrit à ladite section droite.

L'invention concerne également un dispositif de raccordement comprenant un dispositif de perforation comme décrit ci-dessus et un dispositif de réception comportant un élément tubulaire ayant un canal longitudinal central en communication avec l'intérieur d'un conteneur scellé capable de recevoir le dispositif de perforation selon une connexion étanche.

Plus précisément, on atteint les objectifs ci-dessus en réalisant selon l'invention un ensemble de raccordement pour mettre en communication l'intérieur d'un conteneur scellé avec une tubulure d'alimentation par voie entérale comprenant, en combinaison :
**A.** un dispositif de perforation comportant:
   - un élément de perforation formé d'une base tubulaire ayant un évidement ouvert à une première extrémité pour recevoir et maintenir une extrémité de la tubulure d'alimentation, une partie en forme d'aiguille faisant saillie à partir d'une seconde extrémité de ladite base et ayant un canal longitudinal central communiquant avec ledit évidement et débouchant à une extrémité libre de la partie en forme d'aiguille, ladite partie en forme d'aiguille ayant une section droite substantiellement circulaire et au moins une portion située à proximité de ladite base tubulaire dont la section section droite présente au moins une partie en retrait par rapport au cercle circonscrit à ladite section droite; et
   - un élément de blocage;
**B.** un dispositif de réception comportant:
   - une base pourvue d'un orifice central et destinée à raccorder le dispositif de réception au conteneur;
   - un élément tubulaire faisant saillie à partir de la base et comportant un canal longitudinal central communiquant avec l'orifice central de la base et débouchant à une extrémité libre de l'élément tubulaire, ledit canal de l'élément tubulaire étant dimensionné pour recevoir suivant une relation d'étanchéité la partie en forme d'aiguille de l'élément de perforation et ayant au moins une portion située près de ladite extrémité libre de l'élément tubulaire de section droite de forme complémentaire à celle de la partie en forme d'aiguille; et
   - un moyen disposé sur l'élément tubulaire près de ladite extrémité libre de l'élément tubulaire coopérant avec l'élément de blocage du dispositif de perforation pour réaliser une connexion étanche entre le dispositif de perforation et le dispositif de réception mettant en communication l'intérieur du conteneur avec la tubulure d'alimentation.

Dans une réalisation recommandée de l'invention, les portions complémentaires de la partie en forme d'aiguille et de l'élément tubulaire ont une section droite de forme générale ovale.

Dans une réalisation plus particulièrement recommandée ces portions complémentaires comportent au moins un méplat et mieux encore deux méplats diamétralement opposés. Ainsi, comme ces portions complémentaires s'adaptent étroitement l'une à l'autre afin de réaliser une connexion étanche, du fait de la forme ovale ou de la présence du ou des méplats, seul le dispositif de perforation spécifiquement conçu du système d'alimentation par voie entérale peut être introduit dans le dispositif de réception complémentaire en réalisant un raccordement étanche.

Pour encore accroître la sécurité et limiter au maximum le risque d'une connexion erronnée de la part de l'utilisateur, on peut prévoir pour les dispositifs de perforation et de réception de l'ensemble de raccordement une couleur particulière, par exemple verte, distinguant nettement l'ensemble de raccordement d'autres ensembles tels que ceux pour les injections par voie intraveineuse.

Afin d'éviter tout risque de contamination du liquide nutritif contenu, par exemple dans une poche souple, lors du percement de la poche souple pour sa mise en communication avec la tubulure d'alimentation, l'élément de blocage du dispositif de perforation et le moyen complémentaire du dispositif de réception sont conçus de telle sorte que lors de l'introduction initiale par glissement de la partie en forme d'aiguille de l'élément de perforation dans le canal longitudinal central de l'élément tubulaire du dispositif de réception, cette partie en forme d'aiguille se trouve entièrement contenue à l'intérieur du dispositif de réception. Lorsque ensuite l'élément de blocage du dispositif de perforation et le moyen coopérant sur l'élément tubulaire du dispositif de réception sont engagés l'un avec l'autre la partie en forme d'aiguille se déplace vers l'avant dans le canal longitudinal de l'élément tubulaire du dispositif de réception pour faire saillie par l'orifice central de la base de l'élément tubulaire du dispositif de réception jusqu'à une position de blocage finale dans laquelle la partie en forme d'aiguille a perforé la paroi du conteneur, par exemple une poche contenant le liquide nutritif, pour mettre en communication l'intérieur du conteneur avec la tubulure d'alimentation par voie entérale.

Dans une réalisation recommandée, l'élément de blocage est constitué par une bague munie d'un filetage interne et retenue sur l'élément de perforation au moyen d'un rebord transversal annulaire d'extrémité engagé dans une rainure circulaire formée dans la partie en forme d'aiguille de l'élément de perforation près de la base tubulaire de ce dernier, cependant que le moyen coopérant de l'élément tubulaire du dispositif de réception est alors constitué par un filetage externe près de l'extrémité libre de l'élément tubulaire du dispositif de réception. La bague filetée tourne librement dans la rainure de la partie en forme d'aiguille de l'élément de perforation de telle sorte que, lorsque cette bague est vissée sur le filetage complémentaire porté par l'élément tubulaire, elle applique une force au rebord antérieur de la rainure ce qui fait avancer l'élément de perforation dans l'élément tubulaire du dispositif de réception jusqu'à la position de blocage finale. Dans cette position de blocage finale, la partie en forme d'aiguille de l'élément de perforation fait saillie de l'orifice central de la base du dispositif de réception pour percer une paroi du conteneur. Ainsi, le conteneur, par exemple une poche scellée souple, n'est percé et mis en communication avec la tubulure d'alimentation qu'une fois l'ensemble de raccordement mis en place et protégeant l'intérieur du conteneur d'une contamination éventuelle.

Dans un mode de réalisation recommandé, le dispositif de perforation et le dispositif de réception sont réalisés en une matière plastique, par exemple par moulage par injection. On recommande particulièrement comme matière plastique pour le dispositif de perforation une résine ABS qui peut être teintée dans la masse, par exemple en vert, et pour le dispositif de réception du polyéthylène qui également peut être teinté dans la masse, par exemple en vert. De préférence, l'élément de perforation du dispositif de perforation est moulé par injection d'une seule pièce cependant que l'élément de blocage est moulé par injection séparément puis monté à force sur l'élément de perforation. Le dispositif de réception est généralement également moulé par injection d'une seule pièce.

Afin d'éviter toute contamination avant le raccordement, on prévoit un capuchon, généralement en matière plastique (par exemple en polyéthylène) qui s'adapte sur la partie en forme d'aiguille de l'élément de perforation du dispositif de perforation. Bien évidemment, on prévoit également un bouchon, par exemple formé par injection de polyéthylène, qui s'adapte de façon étanche sur l'extrémité libre du dispositif de réception fixé au conteneur, par exemple une poche souple scellée.

L'invention concerne également un procédé de raccordement d'une tubulure d'alimentation par voie entérale à un conteneur rempli d'un liquide nutritif, par exemple une poche souple, pour mettre l'intérieur du conteneur en communication avec la tubulure d'alimentation en utilisant le système de raccordement ci-dessus qui comprend les étapes consistant à:
- insérer par glissement la partie en forme d'aiguille de l'élément de perforation dans le canal longitudinal central du dispositif de réception jusqu'à ce que l'élément de blocage vienne buter contre le moyen coopérant de l'élément tubulaire du dispositif de réception, la partie en forme d'aiguille se trouvant ainsi entièrement contenue à l'intérieur dudit canal; et
- engager l'élément de blocage avec le moyen coopérant du dispositif en réception jusqu'à une position de blocage finale pour avancer la partie en forme d'aiguille et la faire saillir hors de l'orifice central de la base du dispositif de réception, perforant ainsi la paroi du conteneur et réalisant une connexion étanche entre le conteneur et la tubulure d'alimentation.

La suite de la description se réfère aux figures annexées qui représentent respectivement:
- figure 1, une vue éclatée d'une réalisation, selon l'invention, d'un ensemble de raccordement pour un système d'alimentation par voie entérale;
- figure 2, une vue de côté d'une réalisation d'un dispositif de perforation d'un ensemble de raccordement selon la présente invention;
- figure 3, une vue de dessus du dispositif de perforation de la figure 2;
- figure 4, une vue en coupe faite suivant la ligne IV-IV de la figure 2;
- figure 5, une vue de côté d'une réalisation d'un dispositif de réception de l'ensemble de raccordement selon l'invention;
- figure 6, une vue en coupe du dispositif de réception faite selon la ligne VI-VI de la figure 5;
- figure 7, une vue de dessus du dispositif de réception de la figure 5;
- figure 8, une vue de dessous du dispositif de réception de la figure 5; et
- figure 9, une vue en coupe des dispositifs de perforation et de réception des figures 2 et 5 une fois assemblés.

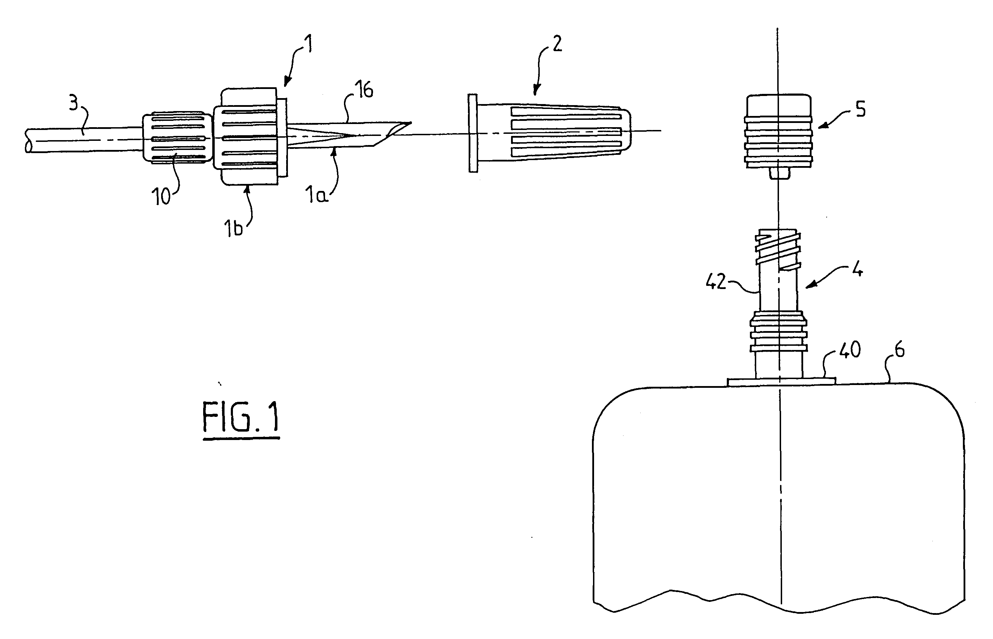

En se référant à la figure 1, on a représenté une vue éclatée d'un ensemble de raccordement pour un système d'alimentation par voie entérale selon l'invention.

D'une manière générale, l'ensemble de raccordement comprend un dispositif de perforation 1 et un dispositif de réception 4. Plus particulièrement, le dispositif de perforation 1 comprend un élément de perforation 1a raccordé à une tubulure d'alimentation 3 et un écrou de blocage 1b maintenu à rotation sur l'élément de perforation 1a. Comme on le voit sur la figure 1, lors du stockage du dispositif de perforation, un capuchon 2 est placé sur l'élément de perforation pour protéger cet élément de toute contamination.

Le dispositif de réception 4 comporte une base 40 et un élément tubulaire 42 faisant saillie de cette base. Le dispositif de réception, comme le montre la figure 1 est fixé par tout moyen approprié, par exemple par collage ou scellage à chaud de la base 40 à un conteneur scellé, par exemple une poche souple en matière plastique 6, contenant un liquide nutritif. Comme pour le dispositif de perforation 1, on prévoit un bouchon 5, par exemple un bouchon cylindrique creux muni d'un téton central qui s'adapte sur l'extrémité libre du dispositif de réception pour en obturer l'ouverture afin d'éviter toute contamination du dispositif de réception pendant le stockage et avant utilisation. De préférence, le dispositif de perforation 1 et le dispositif de réception 4 de l'ensemble de raccordement sont formés par moulage par injection de matière plastique, par exemple une résine ABS et du polyéthylène, respectivement.

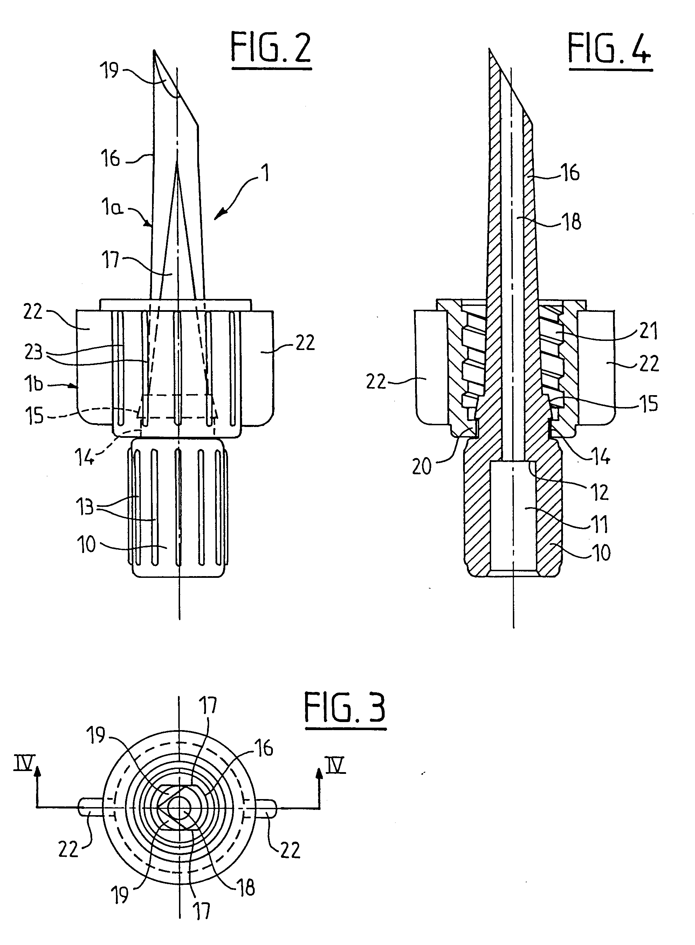

On va maintenant décrire en détail en liaison avec les figures 2 à 4 et 5 à 8, respectivement, les dispositifs de perforation et de réception de l'ensemble de raccordement selon l'invention.

En se référant plus particulièrement aux figures 2 à 4, on a représenté un dispositif de perforation 1 d'un ensemble de raccordement selon l'invention. Comme on le voit sur les figures le dispositif de perforation 1 comprend un élément de perforation 1a d'une seule pièce comportant une base 10 de forme générale cylindrique réunie à une partie en forme d'aiguille 16. La base cylindrique 10 comporte un évidement central 11, de forme généralement cylindrique, qui se termine à son extrémité arrière ou libre par une ouverture chanfreinée. De préférence, la surface latérale externe de la base 10 est pourvue de nervures longitudinales 13 qui renforcent cette base et permettent une meilleure préhension de celle-ci. L'évidement 11 de la base 10 communique, à son extrémité avant avec un canal longitudinal central 18, de forme générale cylindrique, ménagé dans la partie en forme d'aiguille. Le canal longitudinal central 18 de la partie en forme d'aiguille 16, qui est coaxial avec l'évidement 11, a un diamètre inférieur à l'évidement 11, réalisant ainsi une surface annulaire d'appui 12. Une extrémité d'une tubulure souple 3 de diamètre extérieur sensiblement égal au diamètre intérieur de l'évidement 11 est introduite dans cet évidement jusqu'à ce que son bord annulaire d'extrémité vienne s'appuyer contre la surface d'appui annulaire 12. L'extrémité introduite de la tubulure souple peut être fixée de façon permanente à la base cylindrique 10 par tout moyen approprié, par exemple par collage ou par scellage à chaud. De préférence, le canal interne de la tubulure souple est de diamètre généralement égal au diamètre du canal longitudinal central 18 de la partie en forme d'aiguille 16 et se trouve aligné avec ce canal. Le canal longitudinal central 18, de forme générale cylindrique, de la partie en forme d'aiguille 16 débouche à l'extrémité avant ou libre de la partie en forme d'aiguille 16 faisant ainsi communiquer l'évidement cylindrique 11 de la base 10 avec l'extérieur. Comme on le voit sur les figures 2 et 4, l'extrémité avant ou libre de la partie en forme d'aiguille 16 est biseautée de sorte que le canal longitudinal central 18 se termine à cette extrémité libre par un orifice de forme ovale. De plus, l'extrémité libre de la partie en forme d'aiguille 16 est pourvue de deux évidements latéraux symétriques 19 conférant une forme pointue effilée à l'extrémité libre de la partie en forme d'aiguille 16 pour un percement plus facile de la paroi du conteneur.

Selon l'invention, une portion de la partie en forme d'aiguille 16 est pourvue de deux méplats 17 diamétralement opposés qui se prolongent à partir d'une saillie annulaire 15 formée sur la partie en forme d'aiguille 16, à proximité de la base 10 et qui sera décrite plus en détail ci-dessous. Les méplats 17 ont une longueur égale à environ trois quart de la longueur totale de la partie en forme d'aiguille 16. Ces deux méplats 17 diamètralement opposés réalisent dans la partie en forme d'aiguille 16 une portion de forme généralement ovale ou, plus généralement, une portion de la partie en forme d'aiguille 16 ayant une section droite pourvue d'une partie en retrait par rapport à un cercle circonscrit à cette section droite. La longueur ainsi que la forme de ces méplats 17 ne sont pas critiques pour autant qu'ils réalisent sur la partie en forme d'aiguille 16 une portion dont la section droite comporte au moins une partie en retrait par rapport au cercle circonscrit à cette section droite. De même, le nombre de méplats 17 n'est pas critique et on peut le cas échéant, en ménager un seul ou plus de deux. La fonction de ces méplats 17 sera décrite ci-après en liaison avec la description du raccordement du dispositif de perforation 1 avec le dispositif de réception 4.

La partie en forme d'aiguille 16 comme indiqué précédemment comprend une saillie annulaire 15 légèrement écartée de la surface transversale avant de la base cylindrique 10, la saillie annulaire 15 et la paroi transversale avant de la base cylindrique 10 définissant ainsi entre elles une rainure annulaire 14.

Dans une réalisation recommandée, la base cylindrique 10 et la partie en forme d'aiguille 16 sont moulées d'une seule pièce, par exemple par moulage par injection d'une résine ABS.

Comme on le voit plus particulièrement sur les figures 2 et 4, le dispositif de perforation comporte en outre un écrou de blocage 1b. Cet écrou de blocage 1b a la forme générale d'une bague annulaire pourvue à son extrémité arrière d'un rebord transversal annulaire 20 délimitant une ouverture centrale cylindrique d'un diamètre légèrement supérieur au diamètre de la rainure 14 formée dans la partie en forme d'aiguille 16 mais inférieur au diamètre de la saillie annulaire 15 de telle sorte que par emmanchement à force le rebord annulaire 20 vienne se loger dans cette rainure 14 et y soit retenu tout en pouvant tourner autour de l'axe central du dispositif de perforation. Comme on le voit mieux sur la figure 4, l'écrou 1b est muni d'un filetage interne 21. On peut également prévoir sur la paroi latérale externe de l'écrou 1b deux nervures ou oreilles 22 diamétralement opposées afin de permettre une saisie et une rotation plus aisées de l'écrou 1b. On peut de préférence également prévoir des nervures longitudinales 23 de faibles hauteurs sur la surface latérale externe de l'écrou 1b afin de renforcer celui-ci et de rendre plus aisée sa manipulation. La fonction de cet écrou de blocage sera décrite plus complètement lors de la description du raccordement du dispositif de perforation 1 au dispositif de réception 4.

En général, cet écrou est réalisé d'une seule pièce par moulage, par exemple par moulage par injection d'une résine ABS.

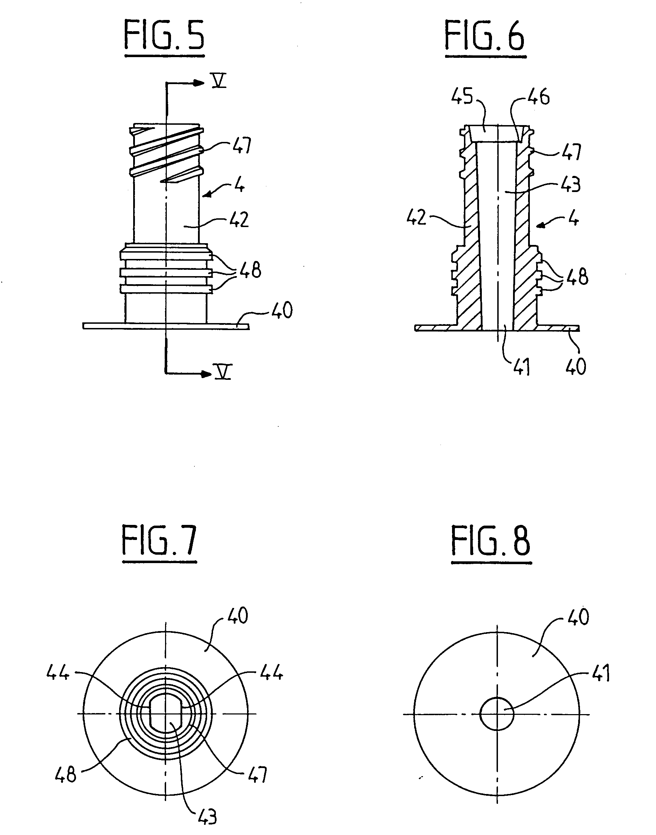

En se référant maintenant aux figures 5 à 8, on a représenté un dispositif de réception 4 d'une réalisation d'un ensemble de raccordement selon l'invention. Comme on le voit sur les figures, le dispositif de raccordement 4 comporte une base 40 se présentant sous la forme d'un disque pourvu d'une ouverture circulaire centrale 41, destiné à être réuni à un conteneur scellé, par exemple une poche souple scellée, par tout moyen approprié tel que collage ou scellage à chaud. Ce dispositif de réception 4 comporte en outre un élément tubulaire 42 faisant saillie de la base 40 et généralement formé intégralement avec celle-ci. L'élément tubulaire 42 comporte un canal central longitudinal 43 communiquant avec l'ouverture central 41 de la base 40 et se terminant à l'extrémité avant ou libre de l'élément tubulaire 42 par un évidement ouvert 45. Le canal longitudinal central 43 du dispositif de réception est de dimension et de forme telles qu'il peut recevoir la partie en forme d'aiguille 16 du dispositif de perforation suivant une relation d'étanchéité. En particulier, dans la réalisation représentée, le canal longitudinal central 43 du dispositif de réception 4 comporte deux méplats 44 diamétralement opposés se prolongeant depuis l'évidement 45 sur une distance égale à environ trois quart de la longueur totale de l'élément tubulaire 42 du dispositif de réception. L'élément tubulaire 42 du dispositif de réception comporte en outre sur sa surface latérale externe, près de son extrémité libre avant, un filetage 47 complémentaire du filetage interne de l'écrou 1b du dispositif de perforation 1. Enfin, on peut également prévoir de façon recommandée une ou plusieurs nervures annulaires (par exemple trois nervures comme représenté sur les figures 5 et 6) sur la surface latérale externe de l'élément tubulaire 42, à proximité de la base 40 du dispositif de réception. Ces nervures accroissent la rigidité de l'élément tubulaire 42.

De manière recommandée, le dispositif de réception est moulé d'une seule pièce, par exemple par moulage par injection de polyéthylène.

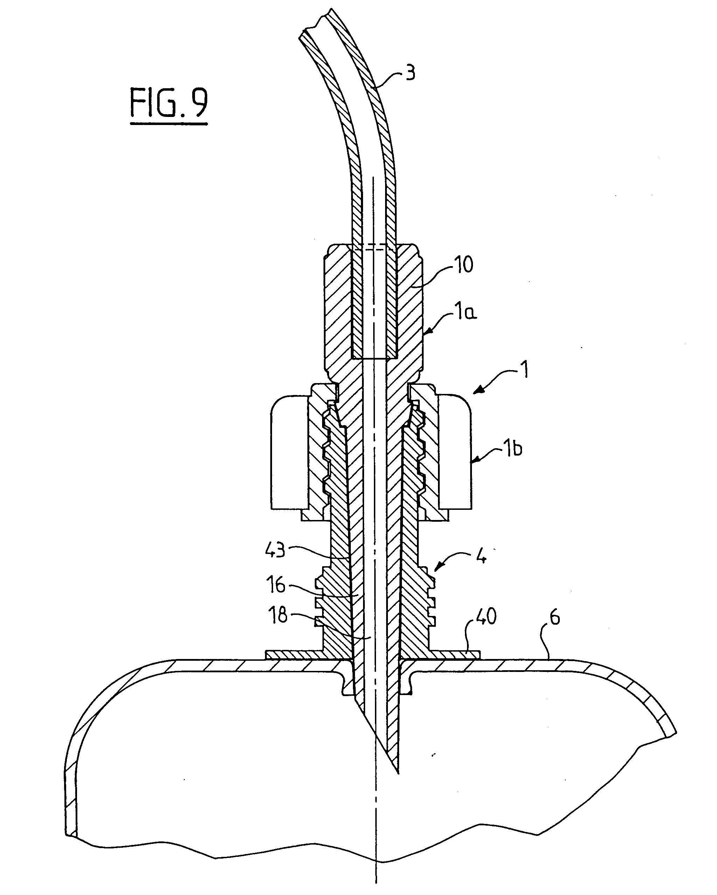

On va maintenant décrire le raccordement du dispositif de perforation et du dispositif de réception 4 de l'ensemble de raccordement selon l'invention en liaison avec les figures 1 et 9.

Une fois le dispositif de perforation 1 raccordé à une tubulure souple 3 et le dispositif de réception 4 réuni à un conteneur, par exemple une poche scellée 6 contenant un liquide nutritif, on enlève le capuchon de protection 2 de la partie en forme d'aiguille 16 du dispositif de perforation et le bouchon 5 de protection du dispositif de réception 4. L'opérateur insère alors la partie en forme d'aiguille 16 du dispositif de perforation 1 dans le canal longitudinal central 43 de l'élément tubulaire 42 du dispositif de réception 4. Du fait de la présence des méplats 17 sur la partie en forme d'aiguille 16 du dispositif de perforation 1 et des méplats correspondants 44 dans le canal longitudinal central 43 de l'élément tubulaire 42 du dispositif de réception 4, l'opérateur ne peut insérer dans ce dispositif de réception 4 que le dispositif de perforation 1 approprié et ne pourrait pas introduire, par exemple, une aiguille de section droite circulaire de diamètre analogue. Ainsi, l'opérateur ne peut, par erreur, introduire dans le dispositif de réception 4 selon l'invention une aiguille de raccordement d'un système d'injection intraveineuse.

Du fait de la présence du filetage 46 sur la paroi latérale externe de l'élément tubulaire 42 du dispositif de réception 4, à proximité de l'extrémité libre ou avant de l'élément tubulaire 42, la partie en forme d'aiguille 16 ne peut être initialement introduite par glissement dans le canal longitudinal 43 de l'élément tubulaire 42 que d'une distance telle qu'elle se trouve entièrement contenue dans l'élément de réception et ne peut pas faire saillie hors de celui-ci par l'orifice central 41 de la base 40 de l'élément de réception, car le filetage interne 21 de l'écrou 1b vient buter contre le filetage externe 47 de l'élément tubulaire 42 du dispositif de réception, empêchant l'opérateur de poursuivre l'introduction par glissement du dispositif de perforation 1. L'opérateur doit alors faire tourner l'écrou 1b pour le visser sur le filetage 47 de l'élément tubulaire 42 du dispositif de réception. En vissant ainsi l'écrou 1b, l'opérateur entraîne en translation l'élément de perforation 1a du fait de la force exercée par le rebord annulaire 20 de l'écrou sur la saillie annulaire 15 de la partie en forme d'aiguille 16 jusqu'à ce que cette saillie annulaire 15 vienne buter contre une surface d'appui annulaire formée à l'interface entre l'évidement annulaire 45 à l'extrémité libre de l'élément tubulaire 42 et le canal longitudinal central 43 de l'élément tubulaire 42 du dispositif de réception 4. Lors du vissage, l'avance de l'élément de perforation la fait saillir l'extrémité biseautée de la partie en forme d'aiguille 16 hors de l'ouverture circulaire 41 de la base 40 de l'élément de réception 4. La partie en forme d'aiguille 16 perfore la paroi scellée de la poche 6 pour mettre en communication l'intérieur de cette poche 6 avec le canal interne de la tubulure souple 3 par l'intermédiaire du canal longitudinal central 18 de la partie en forme d'aiguille 16 communiquant avec le canal interne de la tubulure 3 insérée dans l'évidement 11 de la base cylindrique 10 de l'élément de perforation 1a. Ainsi, la perforation de la poche scellée 6 ne s'effectue que, lorsque l'écrou 1b a été vissé, au moins en partie, sur le filetage externe 47 de l'élément tubulaire 42 du dispositif de réception 4, évitant ainsi un risque de contamination du contenu de la poche 6.

On a ainsi réalisé selon l'invention un système de raccordement pour l'alimentation par voie entérale fiable, étanche, permettant l'utilisation d'un conteneur souple, et évitant tout risque de branchement du conteneur à un système autre que le système d'alimentation par voie entérale.

## Revendications

1. Dispositif de perforation (1) comportant:
- un élément de perforation (la) formé d'une base tubulaire (10) ayant un évidement ouvert (11) à une première extrémité pour recevoir et maintenir une extrémité d'une tubulure d'alimentation (3), une partie en forme d'aiguille (16) faisant saillie à partir d'une seconde extrémité de ladite base et ayant un canal longitudinal central (18) communiquant avec ledit évidement (11) et débouchant à une extrémité libre de la partie en forme d'aiguille, ladite partie en forme d'aiguille (16) ayant une section droite substantiellement circulaire et au moins une portion située à proximité de ladite base tubulaire (10), caractérisé en ce que, la section droite de la portion de la partie en forme d'aiguille situés à proximité de la base comporte au moins une partie en retrait (17) par rapport au cercle circonscrit à ladite section droite.

2. Ensemble de raccordement pour mettre en communication l'intérieur d'un conteneur scellé (6) avec une tubulure d'alimentation par voie entérale comprenant, en combinaison :
**A**. un dispositif de perforation (1) selon la revendication 1; et
**B**. un dispositif de réception (4) comportant un élément tubulaire (42) ayant un canal longitudinal central (43) en communication avec l'intérieur du conteneur scellé (6) capable de recevoir le dispositif de perforation selon une connexion étanche.

3. Ensemble de raccordement pour mettre en communication l'intérieur d'un conteneur scellé (6) avec une tubulure d'alimentation par voie. entérale (3) comprenant, en combinaison :
**A**. un dispositif de perforation (1) selon la revendication 1; et
- un élément de blocage (1b);
**B**. un dispositif de réception (4) comportant :
- une base (40) pourvue d'un orifice central (41) et destinée à raccorder le dispositif de réception (4) au conteneur (6);
- un élément tubulaire (42) faisant saillie à partir de la base (40) et comportant un canal longitudinal central (43) communiquant avec l'orifice central (41) de la base et débouchant à une extrémité libre de l'élément tubulaire, ledit canal (43) de l'élément tubulaire (42) étant dimensionné pour recevoir la partie en forme d'aiguille (16) de l'élément de perforation (1a) et ayant au moins une portion (44) située près de son extrémité libre dont la section droite est complémentaire de ladite portion de la partie en forme d'aiguille (16); et
- un moyen (47) disposé sur l'élément tubulaire(42) près de ladite extrémité libre de l'élément tubulaire coopérant avec l'élément de blocage (1b) dudit dispositif de perforation (1) pour réaliser une connexion étanche entre le dispositif de perforation et le dispositif de réception (4) mettant en communication l'intérieur du conteneur (6) avec la tubulure d'alimentation (3).

4. Ensemble selon la revendication 3 dans lequel ladite portion de la partie en forme d'aiguille (16) a une section droite de forme général ovale.

5. Ensemble selon la revendication 3 dans lequel ladite portion de la partie en forme d'aiguille (16) comporte au moins un méplat (17).

6. Ensemble selon la revendication 5 dans lequel ladite portion de la partie en forme d'aiguille (16) comporte deux méplats (17) diamétralement opposés.

7. Ensemble selon l'une quelconque des revendications 3 à 6 dans lequel la partie en forme d'aiguille (16) comprend en outre une saillie annulaire (15) à proximité de la base (10) définissant avec la base (10) une rainure circulaire (14);
l'élément de blocage (1b) a une extrémité arrière munie d'un rebord transversal annulaire (20), ledit rebord transversal annulaire (20) étant retenu à rotation dans ladite rainure circulaire (14).

8. Ensemble selon la revendication 7 dans lequel ledit élément de blocage est un écrou (1b) pourvu d'un filetage interne (21) et le moyen complémentaire sur l'élément tubulaire (42) est un filetage complémentaire externe (47).

9. Ensemble selon l'une quelconque des revendications 3 à 8, dans lequel la partie en forme d'aiguille (16) de l'élément de perforation (1a) fait saillie hors de l'orifice (41) de la base (40) de l'élément tubulaire (42) du dispositif de réception (4) pour mettre en communication l'intérieur du conteneur (6) avec la tubulure d'alimentation (3) uniquement quand l'élément de blocage (1b) du dispositif de perforation (1) et le moyen coopérant (47) du dispositif de réception (4) sont engagés, au moins en partie, l'un avec l'autre.

10. Ensemble selon l'une quelconque des revendications 3 à 9 dans lequel l'élément de perforation (1a) est réalisé d'une seule pièce en résine ABS, l'élément de blocage (1b) est réalisé d'une seule pièce en résine ABS et le dispositif de réception (4) est réalisé d'une seule pièce en polyéthylène.

11. Procédé de raccordement d'une tubulure (3) d'alimentation par voie entérale à un conteneur (6) rempli d'un liquide nutritif pour mettre en communication l'intérieur du conteneur avec la tubulure d'alimentation au moyen d'un système de raccordement selon l'une quelconque des revendications 3 à 10 comprenant les étapes consistant à:
- insérer par glissement la partie en forme d'aiguille (16) de l'élément de perforation (1a) dans le canal longitudinal central (43) du dispositif de réception (4) jusqu'à ce que l'élément de blocage (1b) vienne buter contre le moyen coopérant (47) de l'élément tubulaire (42) du dispositif de réception (4), la partie en forme d'aiguille (16) se trouvant ainsi entièrement contenue à l'intérieur dudit canal (43); et
- engager l'élément de blocage (1b) avec le moyen coopérant (47) du dispositif de réception pour avancer la partie en forme d'aiguille (16) et la faire saillir hors de l'orifice (41) de la base (40) du dispositif de réception jusqu'à une position de blocage finale, perforant ainsi la paroi du conteneur et réalisant une connexion étanche entre le conteneur et la tubulure d'alimentation.

## Patentansprüche

1. Perforationsvorrichtung (1), die ein Perforationselement (1a) aufweist, das von einer rohrförmigen Basis (10) gebildet wird, die an einem ersten Ende eine offene Aussparung (11) zur Aufnahme und zum Halt eines Endes einer rohrförmigen Ernährungsleitung (3) aufweist, wobei ein nadelförmiger Teil (16) am zweiten Ende der Basis vorsteht und einen zentralen Längskanal (18) besitzt, der mit der Aussparung (11) verbunden ist und am freien Ende des nadelförmigen Teils ausmündet, wobei der nadelförmige Teil (16) einen im wesentlichen kreisförmigen Querschnitt und mindestens einen in Nähe der rohrförmigen Basis (10) gelegenen Bereich besitzt, dadurch gekennzeichnet, daß der Querschnitt des in Nähe der Basis gelegenen Bereichs des nadelförmigen Teils mindestens einen Teil (17) aufweist, der bezüglich des diesem Querschnitt umschriebenen Kreises zurückspringt.

2. Anschlußeinheit zur Verbindung des Inneren eines versiegelten Behälters (6) mit einer rohrförmigen Leitung zur enteralen Ernährung, die in Kombination folgendes umfaßt:
- A. eine Perforationsvorrichtung (1) nach Anspruch 1 und
- B. eine Aufnahmevorrichtung (4), die ein rohrförmiges Element (42) mit einem zentralen Längskanal (43) aufweist, der mit dem Inneren des versiegelten Behälters (6) in Verbindung ist und die Perforationsvorrichtung unter dichter Verbindung aufnehmen kann.

3. Anschlußeinheit zur Verbindung des Inneren eines versiegelten Behälters (6) mit einer rohrförmigen Leitung (3) zur enteralen Ernährung, die in Kombination folgendes umfaßt:
- A. eine Perforationsvorrichtung (1) nach Anspruch 1 und ein Blockierelement (1b),
- B. eine Aufnahmevorrichtung (4), die folgendes umfaßt:
- eine mit einer zentralen Öffnung (41) versehene Basis (40), die dazu bestimmt ist, die Aufnahmevorrichtung (4) mit dem Behälter (6) zu verbinden,
- ein an der Basis (40) vorstehendes rohrförmiges Element (42) mit einem zentralen Längskanal (43), der mit der zentralen Öffnung (41) der Basis verbunden ist und am freien Ende des rohrförmigen Elements ausmündet, wobei der Kanal (43) des rohrförmigen Elements (42) so bemessen ist, daß er den nadelförmigen Teil (16) des Perforationselements (la) aufnehmen kann, und mindestens einen in Nähe seines freien Endes gelegenen Bereich (44) mit einem zu diesem Bereich des nadelförmigen Teils (16) ergänzenden Querschnitt aufweist, und
- eine Einrichtung (47), die an dem rohrförmigen Element (42) in Nähe dessen freien Endes angeordnet ist und mit dem Blockierelement (1b) der Perforationsvorrichtung (1) zusammenwirkt, um eine dichte Verbindung zwischen der Perforationsvorrichtung und der Aufnahmevorrichtung (4) herzustellen, die das Innere des Behälters (6) mit der rohrförmigen Ernährungsleitung (3) verbindet.

4. Einheit nach Anspruch 3, bei der dieser Bereich des nadelförmigen Teils (16) einen Querschnitt von allgemein ovaler Form aufweist.

5. Einheit nach Anspruch 3, bei der dieser Bereich des nadelförmigen Teils (16) mindestens eine Abflachung (17) aufweist.

6. Einheit nach Anspruch 5, bei der dieser Bereich des nadelförmigen Teils (16) zwei diametral einander entgegengesetzte Abflachungen (17) aufweist.

7. Einheit nach einem der Ansprüche 3 bis 6, bei der der nadelförmige Teil (16) ferner in Nähe der Basis (10) einen ringförmigen Vorsprung (15) aufweist, der mit der Basis (10) eine kreisförmige Nut (14) abgrenzt, wobei das Blokkierelement (1b) an seinem hinteren Ende mit einem abgewinkelten ringförmigen Querrand (20) versehen ist, der in der kreisförmigen Nut (14) drehbar zurückgehalten wird.

8. Einheit nach Anspruch 7, bei der das Blockierelement eine mit einem Innengewinde (21) versehene Mutter (1b) und die ergänzende Einrichtung auf dem rohrförmigen Element (42) ein ergänzendes Außengewinde (47) ist.

9. Einheit nach einem der Ansprüche 3 bis 8, bei der der nadelförmige Teil (16) des Perforationselements (1a) aus der Öffnung (41) der Basis (40) des rohrförmigen Elements (42) der Aufnahmevorrichtung (4) herausragt, um das Innere des Behälters (6) mit der rohrförmigen Ernährungsleitung (3) nur dann in Verbindung zu setzen, wenn das Blockierelement (1b) der Perforationsvorrichtung (1) und die zusammenwirkende Einrichtung (47) der Aufnahmevorrichtung (4) zumindest teilweise ineinander eingeführt sind.

10. Einheit nach einem der Ansprüche 3 bis 9, bei der das Perforationselement (la) einstückig aus ABS-Harz hergestellt ist, das Blockierelement (1b) einstückig aus ABS-Harz hergestellt ist und die Aufnahmevorrichtung (4) einstückig aus Polyethylen hergestellt ist.

11. Verfahren zum Anschließen einer rohrförmigen Leitung (3) zur enteralen Ernährung an einen mit einer Nährflüssigkeit gefüllten Behälter (6), um das Innere des Behälters mit der Ernährungsleitung zu verbinden, mit Hilfe eines Anschlußsystems nach einem der Ansprüche 3 bis 10, bestehend aus den folgenden Schritten:
- Einschieben des nadelförmigen Teils (16) des Perforationselements (la) in den zentralen Längskanal (43) der Aufnahmevorrichtung (4), bis das Blockierelement (1b) an der zusammenwirkenden Einrichtung (47) des rohrförmigen Elements (42) der Aufnahmevorrichtung (4) zum Anschlag kommt, wobei der nadelförmige Teil (16) vollständig im Inneren des Kanals (43) enthalten ist, und
- gegenseitiges Ineinander-Einführen des Blockierelements (1b) und der zusammenwirkenden Einrichtung (47) der Aufnahmevorrichtung, um den nadelförmigen Teil (16) vorzubewegen und ihn aus der Öffnung (41) der Basis (40) der Aufnahmevorrichtung bis in eine endgültige Blockierstellung hervortreten zu lassen, so daß die Wand des Behälters perforiert wird und auf diese Weise eine dichte Verbindung zwischen dem Behälter und der rohrförmigen Ernährungsleitung hergestellt wird.

## Claims

1. Perforating device (1) comprising :
- a perforating element (la) formed of a tubular base (10) having an open recess (11) at a first end for receiving and holding one end of an alimentation tube (3), a needle-shaped part (16) projecting from a second end of the said base and having a central longitudinal channel (18) communicating with the said recess (11) and emerging at a free end of the needle-shaped part, the said needle-shaped part (16) having a substantially circular straight section and at least one portion situated close to the said tubular base (10), characterized in that the straight section of the portion of the needle-shaped part situated close to the base includes at least one part (17) recessed with respect to the circle circumscribed about the said straight section.

2. Connecting assembly for putting the interior of a sealed container (6) into communication with an enteral alimentation tube comprising, in combination :
**A**. a perforating device (1) according to claim 1; and
**B**. a receiving device (4) comprising a tubular element (42) having a central longitudinal channel (43)
communicating with the interior of the sealed container (6) capable of receiving the perforating device in a leakproof connection.

3. Connecting assembly for putting the interior of a sealed container (6) into communication with an enteral alimentation tube (3) comprising, in combination :
**A**. a perforating device (1) according to claim 1; and
- a locking element (1b);
**B**. a receiving device (4) comprising :
- a base (40) provided with a central orifice (41) and designed to connect the receiving device (4) to the container (6);
- a tubular element (42) projecting from the base (40) and including a central longitudinal channel (43) communicating with the central orifice (41) of the base and emerging at a free end of the tubular element, the said channel (43) of the tubular element (42) being dimensioned so as to receive the needle-shaped part (16) of the perforating element (1a) and having at least one portion (44) situated close to its free end, of which the straight section is complementary to the said portion of the needle-shaped part (16); and
- a means (47) positioned on the tubular element (42) close to the said free end of the tubular element cooperating with the locking element (1b) of the said perforating device (1) so as to provide a leakproof connection between the perforating device and the receiving device (4), putting the interior of the container (6) into communication with the alimentation tube (3).

4. Assembly according to claim 3, wherein the said portion of the needle-shaped part (16) has a straight section with a generally oval shape.

5. Assembly according to claim 3, wherein the said portion of the needle-shaped part (16) includes at least one flat part (17).

6. Assembly according to claim 3, wherein the said portion of the needle-shaped part (16) includes two diametrically opposed flat parts (17).

7. Assembly according to any one of claims 3 to 6, wherein the needle-shaped part (16) additionally includes an annular projection (15) close to the base (10) defining with the base (10) a circular groove (14);
the locking element (1b) has a rear end provided with an annular transverse rim (20), the said annular transverse rim (20) being retained in rotation in the said circular groove (14).

8. Assembly according to claim 7, wherein the said locking element is a nut (1b) provided with an internal thread (21) and the complementary means on the tubular element (42) is a complementary external thread (47).

9. Assembly according to any one of claims 3 to 8, wherein the needle-shaped part (16) of the perforating element (1a) projects outside the orifice (41) of the base (40) of the tubular element (42) of the receiving device (4) so as to put the interior of the container (6) into communication with the alimentation tube (3) only when the locking element (1b) of the perforating device (1) and the cooperating means (47) of the receiving device (4) are engaged, at least partly, with each other.

10. Assembly according to any one of claims 3 to 9, wherein the perforating element (1a) is made of a single piece of ABS resin, the locking element (1b) is made of a single piece of ABS resin and the receiving device (4) is made of a single piece of polyethylene.

11. Procedure for connecting an enteral alimentation tube (3) to a container (6) filled with a nutrient liquid for putting the interior of the container into communication with the alimentation tube by means of a connecting system according to any one of claims 3 to 10, comprising steps consisting of :
- inserting by sliding the needle-shaped part (16) of the perforating element (1a) into the central longitudinal channel (43) of the receiving device (4) until the locking element (1b) butts up against the cooperating means (47) of the tubular element (42) of the receiving device (4), the needle-shaped part (16) being in this way entirely contained inside the said channel (43); and
- engaging the locking element (1b) with the cooperating means (47) of the receiving device in order to move the needle-shaped part (16) forward and to cause it to project outside the orifice (41) of the base (40) of the receiving device as far as a final locking position, perforating in this way the wall of the container and forming a leakproof connection between the container and the alimentation tube.
